# EUROPEAN PATENT SPECIFICATION

(11) **EP 2 627 634 B1**
(45) Date of publication and mention of the grant of the patent: **26.08.2015**
(21) Application number: 11781740.3
(22) Date of filing: 13.10.2011
(51) Int. Cl.: C07D 209/94, C07D 403/12, C07D 405/12, C07D 487/08, C07K 1/00, C07K 14/00, C07K 17/14, C07K 1/14, C07K 1/22

(54) **A NEW DIMEDON DERIVATIVE AND A METHOD FOR THE PURIFICATION OF PNA AND PEPTIDE OLIGOMERS**
NEUES DIMEDONDERIVAT UND VERFAHREN ZUR REINIGUNG VON PNA UND PEPTIDOLIGOMEREN
NOUVEAU DÉRIVÉ DE DIMÉDON ET PROCÉDÉ DE PURIFICATION D'APN ET D'OLIGOMÈRES PEPTIDIQUES

(30) Priority: 13.10.2010 EP 10187366
(43) Date of publication of application: 21.08.2013
(73) Proprietor: Deutsches Krebsforschungszentrum, 69120 Heidelberg (DE)
(72) Inventor: JACOB, Anette, 69221 Dossenheim (DE); HOHEISEL, Jörg, 69168 Wiesloch (DE); DAUBER, Marc, 68309 Mannheim (DE); WIESSLER, Manfred, 67227 Frankenthal (DE); LORENZ, Peter, 69221 Dossenheim (DE); FLEISCHHACKER, Heinz, 69221 Dossenheim (DE); KLIEM, Hans-Christian, 64646 Heppenheim (DE)
(74) Representative: Schüssler, Andrea
(86) International application number: PCT/EP2011/005137
(87) International publication number: WO 2012/048877

(56) References cited:
- WO-A1-2005/012228
- WO-A2-2006/003094
- LIN C T ET AL: "Synthesis of novel triketone-based acidichromic colorants", TETRAHEDRON LETTERS, ELSEVIER, AMSTERDAM, NL, vol. 46, no. 30, 25 July 2005 (2005-07-25) , pages 5033-5037, XP025385643, ISSN: 0040-4039, DOI: DOI:10.1016/J.TETLET.2005.05.070 [retrieved on 2005-07-25]
- DATABASE CA [Online] CHEMICAL ABSTRACTS SERVICE, COLUMBUS, OHIO, US; 1995, MIKHAL'CHUK, A. L. ET AL: "Enamino diketones. Reaction of 2-(1-aminoethylidene)cyclohexane-1,3-dione s with N,N-dialkylformamide dialkyl acetals", XP002625989, retrieved from STN Database accession no. 1995:789068 & MIKHAL'CHUK, A. L. ET AL: "Enamino diketones. Reaction of 2-(1-aminoethylidene)cyclohexane-1,3-dione s with N,N-dialkylformamide dialkyl acetals", ZHURNAL ORGANICHESKOI KHIMII , 31(1), 151 CODEN: ZORKAE; ISSN: 0514-7492, 1995,
- DATABASE CA [Online] CHEMICAL ABSTRACTS SERVICE, COLUMBUS, OHIO, US; 2008, VALLDOSERA, MAGDALENA ET AL: "Comparative Study of Chemical Approaches to the Solid-Phase Synthesis of a Tumor-Seeking .alpha.-MSH Analogue", XP002625991, retrieved from STN Database accession no. 2008:1176860 -& VALLDOSERA, MAGDALENA ET AL: "Comparative Study of Chemical Approaches to the Solid-Phase Synthesis of a Tumor-Seeking .alpha.-MSH Analogue", INTERNATIONAL JOURNAL OF PEPTIDE RESEARCH AND THERAPEUTICS , 14(3), 273-281 CODEN: IJPRFC; ISSN: 1573-3149, 2008, XP002625992,
- DATABASE CA [Online] CHEMICAL ABSTRACTS SERVICE, COLUMBUS, OHIO, US; 2009, DIXON, MARK J. ET AL: "Solid-phase synthesis of cyclic peptide chitinase inhibitors: SAR of the argifin scaffold", XP002625993, retrieved from STN Database accession no. 2008:1535891 -& DIXON, MARK J. ET AL: "Solid-phase synthesis of cyclic peptide chitinase inhibitors: SAR of the argifin scaffold", ORGANIC & BIOMOLECULAR CHEMISTRY , 7(2), 259-268 CODEN: OBCRAK; ISSN: 1477-0520, 2009, XP002625994,
- DUMY ET AL: TETRAHEDRON LETT., vol. 36, no. 8, 1995, pages 1255-1258, XP002625995,
- BRANDT ET AL: NUCLEIC ACIDS RES., vol. 31, no. 19, 2003, page E119, XP002625990, cited in the application
- "Oligonucleotide synthesis", , Retrieved from the Internet: URL:en.wikipedia.org/wiki/Oligonucleotide_ synthesis [retrieved on 2013-11-22]

## Description

The present invention concerns a new dimedon derivative and a method for the purification of PNA and peptide oligomers.

Valldosera et al. (INTERNATIONAL JOURNAL OF PEPTIDE RESEARCH AND THERAPEUTICS, 14(3), 273-281, 2008) disclose a comparative study of chemical approaches to the Solid-Phase Synthesis of a tumor-seeking α-MSH analogue.

Dixon et al (ORGANIC & BIOMOLECULAR CHEMISTRY, 7(2), 259-268, 2009) disclose a solid-phase synthesis of cyclic peptide chitinase inhibitors.

Dumy et al (TETRAHEDRON LETT., vol. 36, no. 8, 1995, pages 1255-1258) disclose the synthesis of cyclic peptides as regioselectively addressable functionalized templates (RAFT).

Ching-Tsan Lin et al., (Tetrahedron Letters 46, (2005), pp. 5033-5037) concern the synthesis of triketone-based acidichromic colorants.

Database CA, Database accession No. : AN 1995:789068 describes the reaction of 2-(1-aminoethylidene)cyclohexane-1,3-diones with N,N-dialkylformamide dialkyl acetals.

WO 2005/012228 A1 describes O-coordinated metal chelates and their use in optical recording media having high storage capacity.

WO 2006/003094 A2 describes merocyanine derivatives that are useful in cosmetic and pharmaceutical preparations.

DNA oligonucleotide microarray technology is applied in biological and biomedical research on a routine basis. Various biological aspects can be studied with this technology of which transcriptional profiling (Schena, M. et al., 1995, Science, 270: 467-470) and detection of single nucleotide polymorphisms (SNPs) (Guo, Z. et al., 1994, Nucleic Acids Res., 22: 5456-5465; Dawson, E. et al., 2002, Nature, 418: 544-548) are currently the most widely performed analyses. However, DNA oligonucleotide microarrays have some shortcomings when dealing with samples of a high complexity (Dubiley, S. et al., 1999, Nucleic Acids Res., 27: e19; Syvänen, A.-C., 1999, Hum. Mutat., 13: 1-10).

For this reason, modern biological applications utilize other oligomers, such as peptides or peptide nucleic acids (PNAs), as probe molecules. For example, PNA oligomers are synthetic DNA mimics with an amide backbone (Nielsen, P. E. et al., 1991, Science, 254: 1497-1500; Egholm, M. et al., 1993, Nature, 365: 566-568). Compared to DNA oligonucleotides, PNAs are stable under acidic conditions and resistant to nucleases as well as to proteases (Demidov, V. V. et al., 1994, Biochem. Pharmacol., 48: 1310-1313). Their neutral backbone increases the binding strength to complementary DNA compared to the stability of the respective DNA duplex (Wit-tung, P. et al., 1994, Nature, 368: 561-563; Ray, A. and Norden, B., 2000, FASEB J., 14: 1041-1060). Thus, the application of oligomers, such as peptides or PNAs, as array probe molecules offer an alternative way with superior performance (Weiler, J. et al., 1997, Nucleic Acids Res., 25: 2792-2799).

However, for modern molecular biological applications, such as DNA- or PNA-microarrays or libraries of peptides or sugars, the quality and purity of the oligomers are the most critical prerequisites. To date, standard methods are used for the purification of oligomers subsequent to their synthesis, such as the high-performance-liquid-chromatography (HPLC). The procedure of HPLC, however, is very labour intensive, time-consuming and expensive, i.e. HPLC is neither adapted for high-throughput oligomer synthesis nor for high-throughput oligomer purification. Thus, a method has to be developed for high-throughput applications for the synthesis and purification of thousands of different oligomers. There is a need for an alternative way to purify synthetically produced full-length oligomers, such as PNAs or peptides in parallel.

To date, several strategies have been developed to avoid labour intensive, time-consuming and expensive synthesis and purification of oligomers. For example, different coupling ways for achieving a selective covalent binding of synthesized full-length PNA products to a microarray solid support have been analysed in detail. For example, succinimidyl-activated slides in connection with unmodified oligomers with a primary amino group (H₂N-PNA-CONH₂); maleimide-activated or gold-coated slides and PNAs, to which a cysteine was added during the last synthesis cycle (Cys-PNA-CONH₂); and streptavidin-coated slides and PNAs with an N-terminal biotin (biotin-PNA-CONH₂) (Brandt, O. et al., 2003, Nucleic Acids Res., 31: e119). By means of these methods, the full-length PNAs have been attached to the micorarray solid support, whereas all shorter PNA derivatives resulting from incomplete condensation during PNA synthesis, however, did not bind. The shorter derivatives have been capped prior to the following extension reaction, caused by the acetylation of their amino ends, which inhibits their binding. Thus, the full-length PNA products were purified since the shorter derivatives were washed away. All above mentioned purification schemes by oligomer bioconjugation produced good results, whereas maleimide surfaces in combination with thiol-modified PNAs were the overall best choice.

However, all of these methods have the disadvantage that they do not work with peptides and PNA good enough. With peptides and PNA the problem is that they may contain NH₂ and SH groups in their side chains, and, thus, also incomplete sequences are able to bind to the support and cannot be traceless removed to further react in solutions. This may have advantages for the chip production but not for the purification of PNA and peptide molecules for assays in solution. In addition, many known methods work only in buffered aqueous systems but not in organic solutions.

Thus, the problem to be solved by the present invention is the provision of a compound that helps to establish an inexpensive and effective method for the purification of oligomers bound to a solid support and, if desired, may be easily cleavable therefrom. These solid supports shall be suitable for high-throughput applications, such as PNA-microarrays and library screening.

The problem is solved by the subject-matter of the present claims 1 and 2. Preferred embodiments are the subject matter of the dependent claims.

The inventors have recognized that a new traceless linker is helpful for the purification of many biological molecules. This new traceless linker is a dimedon (= 5,5-dimethylcyclohexane-1,3-dion) derivative. The dimedon derivatives of the present invention are selected from the group consisting of:

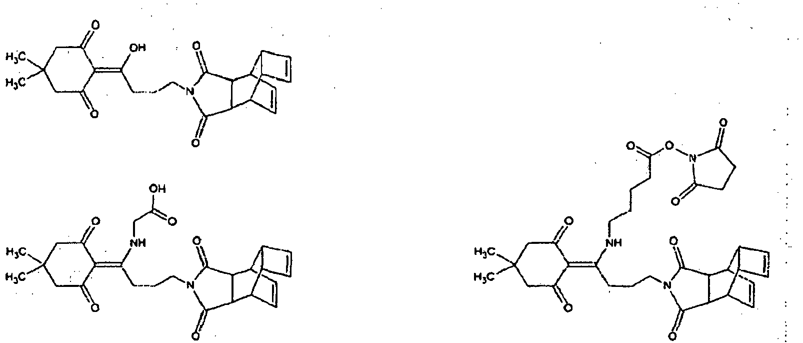

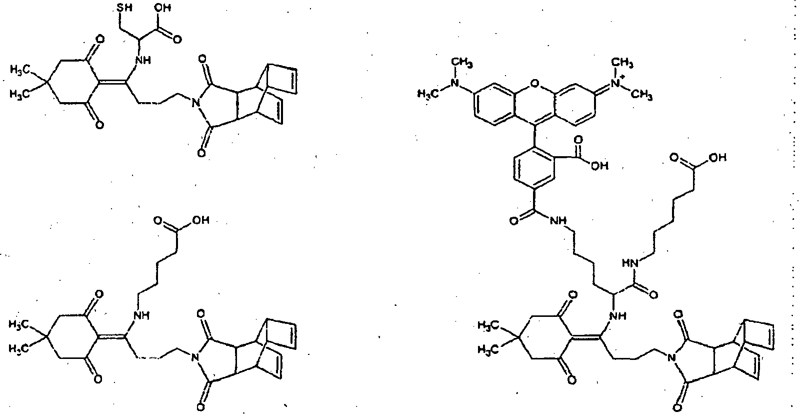

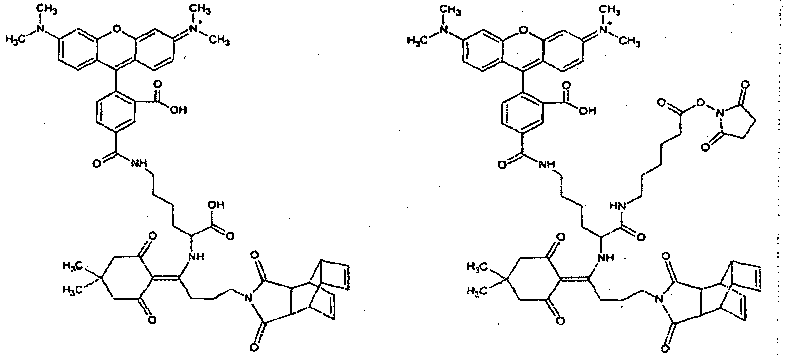

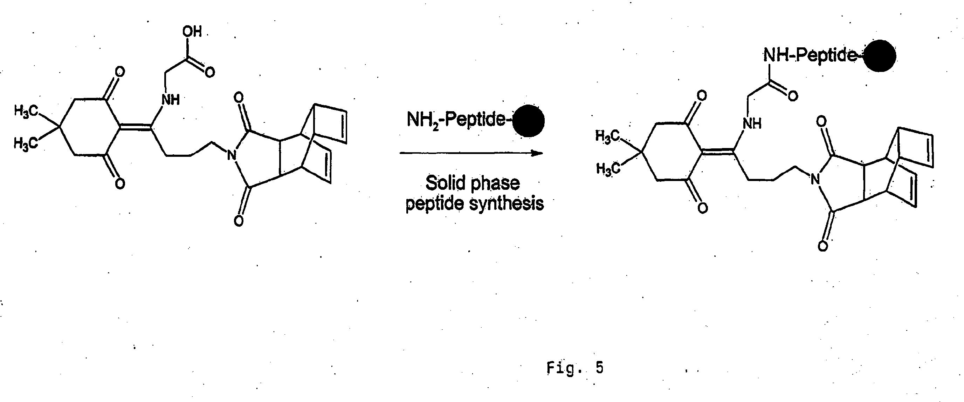

The dimedon derivatives of the present invention are particularly useful for the purification of compounds like peptides, PNAs, proteins, sugars, carbohydrate, DNA and polymers. In this regard it is preferred that the dimedon derivative is coupled as the last component in the solid phase synthesis of the compounds to facilitate their purification. The inventors have recognized that it is advantageous for the reaction conditions of the purification step if glycine is contained in the dimedone derivative (c.f. Fig. 5).

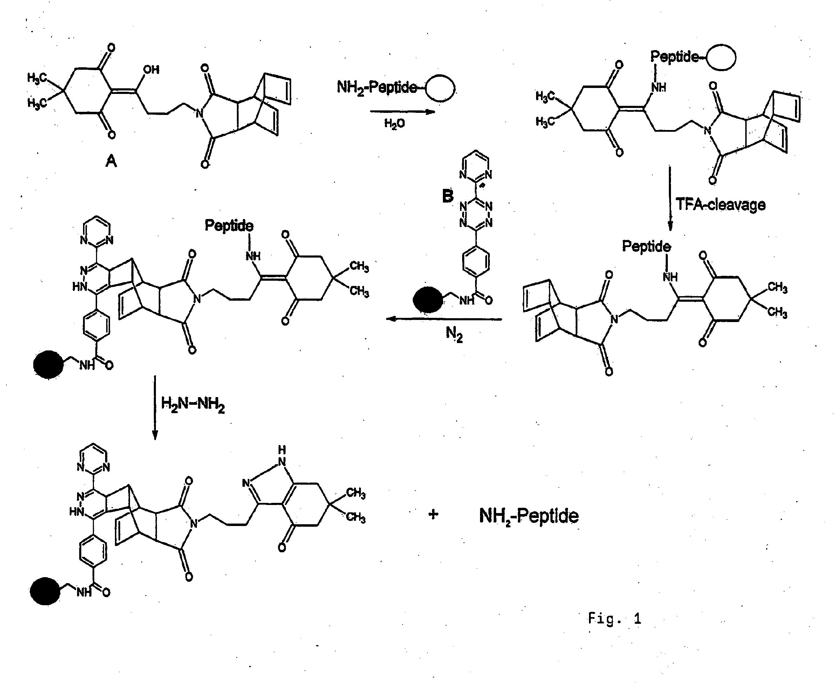

In the following the purification of peptides/PNAs is described where the above described dimedon derivative is used as a cleavable purification-"tag". In this regard reference is made to Fig. 1. This figure has to be understood as exemplary and shall not be construed as limiting the shown reaction to peptides only. All above mentioned compounds may undergo similar reactions.

Refering to Fig. 1, a dimedon derivative carrying a dienophil residue is coupled to a peptide that is bound to a solid phase. After cleavage of the peptide from the solid phase (i.e. the synthesis resin), the crude product is reacted with a dien (e.g. a tetrazin derivative) bound to a second solid support. In this inverse electron demand Diels Alder reaction only full length products with the dienophile moiety are able to bind to the dienes and all shorter and incomplete products without dienophile moiety can be removed. If desired, at the end a purified peptide can be cleaved from the solid support and can be used for any desired purpose.

In a preferred embodiment the dimedon derivative contains a NHS ester that can be coupled to the free amino function in any molecule (e.g. proteins, DNA, peptides, sugars, PNAs) to be tagged, then binding of the molecule via inverse Diels-Alder reaction to a solid phase and removing of the no-tagged molecules (similar to Fig. 1). Then the purified tagged molecule may be cleaved from the solid phase and may be used for any suitable purpose. This embodiment has the advantage that the tagging of the molecules can be made in such a way that statistically only one dye is bound per molecule (possible application: single molecule detection) and all non-tagged molecules may be removed after the binding. This provides only tagged products.

In general, the reaction of the present invention is based on an inverse electron demand Diels Alder (DA) reaction on a solid support, wherein the solid support is modified by carrying a diene or dienophile as a first binding partner on its surface. This surface-immobilized diene or dienophile is then reacted via an inverse demand DA with a peptide oligomer carrying an above described dimedon derivative comprising a dienophile or diene as a second binding partner, i.e. depending whether the solid support carries a diene or dienophile as binding partner, the oligomer has to carry the corresponding binding partner. All definitions given with respect to the above described dimedon derivative also apply with regard to the method of the present invention.

The diene (D) is selected from the group consisting of 1,2,4,5-tetrazines, 1,2,4-triazines and 1,2-diazines, wherein these dienes can be mono- or polysubstituted with electron-withdrawing groups. The electron-withdrawing groups can be selected from the group consisting of:
COOR₃, preferably COOH;
C(O)N(R₃)₂;
CX₃, wherein X is halogen, preferably CF₃;
halogen, selected from the group consisting of F, Cl, Br or I;
CN;
SO₂-R₃ or SO₃-R₃;

Preferred dienes are any type of ester of 1,2,4,5-tetrazines, 1,2,4-triazines and 1,2-diazines and of their derivatives: e.g. tetrazine-monoamide; tetrazine-diamide; amide of tetrazine-3-trifluoromethyl-6-carboxylic acid; mono-, di- or triamides of triazine-tricarboxylic acid; 3-carboxyamide-5,6-bis-trifluor-methyl-triazine, 1,2-diazine-3,6-dicarboxylic acid-amide and homologues thereof with a ethyl- or propyl-group instead of the methyl-group. The above mentioned tetrazines may be produced by oxidation of the corresponding dihydro compounds, which are amenable via the dihydro tetrazine dicarboxylic acid ester, wherein the dihydro tetrazine dicarboxylic acid ester may be produced via two steps by means of the purchasable diazoesters. The preparation of the dienes is well known in the art and is within the knowledge of a chemist.

Various dienophiles suitable for use in the inverse electron demand Diels Alder (DA) reactions are known in the art and/or can be selected by the ordinarily skilled artisan. In general, a dienophile will be selected according to criteria such as: 1) the reactivity of the dienophile, 2) the reactivity of the diene, 3) the desired structure of the inverse electron demand DA product, and 4) the availability of the dienophile (e.g. commercial availability or synthetic accessibility).

As dienophile, one terminal olefinic double bond without any activation is sufficient. However, double bonds, which are integrated in carbocyclic ring systems, may also be utilized, wherein triple bonds in ring systems greater than 7-membered rings may also react as inverse dienophiles. Furthermore, isolated olefinic double or triple bonds in a linear or branched hydrocarbon chain, which may contain hetero atoms such as N, O, S or Si, are also utilizable.

Preferred dienophiles are acids and anhydrides and their functionalized imide- and amide-derivatives and their reduction products and the associated ester and their substitution and reduction products, which contain a strained or a terminal double bond, e.g. exo- or endo-norbornene-dicarboxylic acid anhydride, cyclobutene-dicarboxylic acid anhydride, cyclohexene-dicarboxylic acid anhydride, and the easily available COT-MSA adduct with two reactive double bonds, i.e. a cyclobutene ring and a cyclohexene ring. That applies to the 2-allyl-2-propargyl-malonester too. Further preferred dienophiles are dehydroproline, allyl-proline, allylmalone ester, allylga-lactose, allyl-silsesquioxane and all compounds, which contain either an allyl-, butenyl- or pentenyl-group. The dienophile can be mono- or polysubstituted with functional groups, wherein the functional groups may be selected from the group consisting of alkyl chains (C₂ - C₂₀, preferably methyl, ethyl, iso-propyl, tert.-butyl etc., where appropriate substituted with halogen), OH, SH, halogens, aryl-, carboxyl-, carbonyl-, nitro-, nitril-, carboxyamido-, keto-, sulfoxide-, sulfone-, sulfonic acid-, sulfide-, sulfate-, phosphoric acid- or amino-groups, which are directly bound via alkyl residues. The dienophile may also contain aromatic or heterocyclic residues, which may be selected from the group consisting of: phenyl-, thienyl-, thiophenyl-, furyl-, furanyl-, cyclopentadieneyl-, pyranyl-, pyrrolyl-, imidazolyl, pyrazolyl-, pyridyl-, pyrimidinyl-, pyrazinyl-, pyridazinyl-, thiazolyl-, oxazolyl-, indolyl-, furazannyl-, pyrrolinyl-, imidazolinyl-, pyrazolinyl-, thiazolinyl-, triazolyl-, tetrazolyl- and position isomers of the heteroatom(s), which may comprise these groups, a residue consisting of carbocyclic condensed rings, for example naphthyl group or phenanthrene group, a residue consisting of heterocyclic condensed rings, for example benzofuranyl, benzothienyl, benzimidazolyl, benzothiazolyl, naphtho[2,3-b]thienyl, thianthrenyl, isobenzofuranyl, chromenyl, xanthenyl, phenoxathionyl, indolizinyl, isoindolyl, 3H-indolyl, indolyl, indazolyl, purinyl, chinolizinyl, isochinolyl, chinolyl, phthalzinyl, naphthyridinyl, chinoxalinyl, chinazolinyl, chinolinyl, pteridinyl, carbazolyl, ß-carbolinyl, cinnolinyl, acridinyl, phenazinyl, phenothiazinyl, phenoxazinyl, indolinyl, isoindolinyl, imidazopyridyl, imidazopyridmidinyl or the polycyclic condensed systems consisting of heterocyclic monocycles, for example as defined above, for example as thionaphthenyl, furo[2,3-b]pyrrol or thieno[2,3-b]furan, and in particular the phenyl-, furylgroups, as 2-furyl, imidazolyl, as 2-imidazolyl, pyridyl, as 2-pyridyl, 3-pyridyl, 4-pyridyl, pyrimidinyl, as pyridmid-2-yl, thiazolyl, as thiazol-2-yl, thiazolinyl, as thiazolin-2-yl, triazolyl, as triazolyl-2-yl, tetrazolyl, as tetrazol-2-yl, benzimidazolyl, as benzimidazol-2-yl, benzothiazolyl, benzothiazol-2-yl, purinyl, as purin-7-yl or chinolyl, as 4-chinolyl.

A "dye" may be a fluorescent, phosphorescent or chemiluminescent dye. In particular, a xanthene dye (e.g. fluorescein), ethidiumbromide, fluorescent protein (e.g. GFP, YFP, RFP), rhodamine, cumarines, DAPI, porphyrins, luminol, diphenyloxalate, biotin.

Since generally during the oligomer synthesis shorter sequences occur which normally have to be removed before binding the oligomer to a solid support, the method of the present invention has the advantage that only those oligomers bind which carry the diene or dienophile (wherein the diene or dienophile is preferably bound at the end of the oligomer) and shorter incomplete sequences have not to be removed before carrying out the inverse electron demand Diels Alder reaction on the solid support because these incomplete sequences cannot bind. This means that the diene- or dienophile-modified oligomer preparation can be used as a crude preparation without any further purification. After the inverse electron demand DA reaction. i.e. after the modified oligomer has been bound to the modified solid support, these unbound incomplete oligomers, residual monomer elements and other contaminations may be washed away. The solid supports prepared according to the present invention, i.e. on which a modified oligomer has been immobilized, are particularly useful for microarrays, e.g.:
- PNA-microarrays which may be utilized for the analysis and detection of mutations, expression analysis, purification of RNAs and DNAs with "PNA capture probes" (e.g. Ørum, H., 1999, Curr. Iss. Mol. Biol., 1: 105-110), for the specific detection of microorganisms or for the detection of binding domains of protein/DNA interactions,
- Peptide-microarrays which may be utilized for the analysis of enzyme and protein function, analysis of the binding site of antibodies and drug discovery or the manufacture of peptide libraries and the detection of e.g. phosphorylation sites in peptides or proteins,
- Protein-microarrays which enable to detect interactions between proteins as well as protein and DNA or RNA.
- In general, oligomer-arrays have attracted wide interest as tools for discovering biochemical interactions. Several methods have been reported for the preparation of oligomer arrays, such as protein arrays (e.g. Maercker, C., 2005, Bioscience Rep., 25: 57-70), peptide arrays (e.g. Min, D.-H. and M. Mrksich, 2004, Curr. Opin. Chem. Biol., 8: 554-558) or PNA arrays (e.g. Weiler, J. et al., 1997, Nucleic Acids Res., 25: 2792-2799). The spotted molecules allow the detection of e.g. interactions between enzymes and substrates, between antibodies and antigens, between receptors and hormones, between nucleic acids and transcription factors. Even modifications of macromolecules are possible to detect. Furthermore, in combination with compound libraries, it is possible to detect and identify new active drug lead molecules. In further steps, these substances can be brought into solution and used for functional studies. By compatible peptide chemistry, drug candidates can be changed into active agents.

The term "(peptide) oligomer" refers to any biomolecule with an amide backbone, which consists of a limited number of monomer units, wherein the number is typically in the range of 5 - 100 monomers, preferably in the range of 6 - 20 monomers. Preferred oligomers are short, single stranded analogs of nucleic acids, such as peptide nucleic acids (PNAs). It can also refer to peptides, formed from the linking of amino acids in a defined order. More precisely, the term "peptide" refers to an oligopeptide consisting of 2 - 10 amino acids, such as di- or tripeptides. Furthermore, the term oligomer can also refer to a "protein complex" made of two or more subunits of any length. In case that the complex is made of different protein subunits, said complex is termed a hetero-oligomer or heteromer. When only one type of protein subunit is used in the complex, it is termed a homo-oligomer or homomer.

The term "modified (peptide) oligomer" refers to an oligomer as defined above, wherein the oligomer has been bound to at least one dimedon derivative of the present invention carrying a diene or dienophile. The diene or dienophil is preferably the last element of this reaction product.

The term "solid support" and "solid phase" refers to any type of solid or insoluble moiety suitable for the immobilization of an oligomer, and further reaction or synthesis and purification of the immobilized oligomer, e.g. commonly utilized carriers for the production of biochip/arrays, e.g. glassware, films or membranes (e.g. of PP, PE, nylon, cellulose, cellulose mixed ester, PA), semiconductors, nanotubes, chitin, chitosan, semipermeable membranes, simplex-membranes, ceramics, hybridpolymers or nanocomposites. In a preferred embodiment, the solid support is a polymeric support. In certain embodiments, the solid support is in the form of discrete particles, e.g. beads.

The term "modified solid support" refers to a solid support as defined above, wherein at least one diene or dienophile has been immobilized at the surface of the solid support. This immobilization of the diene or dienophile on the solid support may be through a direct bond, a linker or spacer arm. A linker arm can conveniently be attached to, and, if necessary, cleaved from, the diene or dienophile and/or the solid support, preferably under mild conditions, to permit the easy immobilization of a diene or dienophile on the solid support and the ready detachment of a product from the solid support, without causing undesirable side reactions. A variety of linkers which may find use in the invention are known in the art. Examples are polyethyleneglycol (PEG) linker, methylbenzhydrylamine (MBHA) linker or Rink linker. If a direct bond of the diene or dienophile on the solid support is preferred, a "coupling reagent", such as 1-hydroxy-7-azabenzotriazole (HOAt) or *N,N'-*diisopropylcarbodiimide (DIC) and the like, is preferably used.

The method of the present invention involves the inverse electron demand DA reaction of a diene with a dienophile, wherein either the diene or the dienophile is immobilized on the solid support. The inverse electron demand DA reaction requires an electron-poor diene and an electron-rich dienophile. Appropriate choice of diene and dienophile can, thus, affect the product of the reaction and should be chosen to favour the desired product. The criteria to choose the diene/dienophile are those as mentioned before and the diene/dienophile are those as mentioned before.

The inverse electron demand DA reaction is a standard procedure in the organic chemistry and the reaction conditions are known by the skilled artisan. In general, it is desirable that the reactions are run using mild conditions that will not adversely affect the diene, the dienophile, any intermediates, or the product. For example, the reaction temperature influences the speed of the reaction, as well as the stability of the reactants and catalyst. The reactions will usually run at temperatures in the range of 20°C to 100°C.

Furthermore, the inverse electron demand DA reaction of the present invention can be performed in any type of solvent, e.g. in solution or suspension. Suitable inert solvents include ethers such as tetrahydrofurane, dioxane, diethylether and the like, halogenated solvents such as dichloromethane, dichloroethane, trifluoroacetic acid (TFA) and the like, polar aprotic solvents such as dimethylformamide (DMF), dimethysulfoxide (DMSO), acetonitrile (ACN) and the like, alcohols such as methanol, ethanol and the like, and water or a combination of two or more solvents.

In certain embodiments, it is preferable to perform the reactions under an inert atmosphere of a gas such as nitrogen or argon.

In a preferred embodiment, the reaction conditions are selected to permit isolation or simultaneous synthesis and purification of the inverse electron demand DA adduct, or a product resulting from rearrangement of the above mentioned adduct.

It is known that the reaction rate or stereochemical outcome of certain DA reactions can be affected by the use of catalysts such as Lewis acid catalysts, including, e.g. boron trifluoride etherate, aluminium chloride, SnCl₄ and the like. Accordingly, the present invention contemplates the use of catalysts in the inverse electron demand DA reaction, where appropriate.

In a preferred embodiment the (peptide) oligomer may be cleaved with aqueous hydrazine from the Diels Alder product that is bound to the solid support (c.f. last step of Fig. 1). This provides a purified oligomer since the bond between the oligomer and the dimedon derivative is easily cleavable. This easy removal of the dimedone tag is one of the greatest advantages of the present invention when purified oligomers are to be provided.

In general, the invention provides a method for the simultaneous synthesis and purification of oligomers by their immobilization on a solid support for high-throughput applications such as PNA-microarrays and libraries of peptides. The methods involve the synthesis of a dimedon derivative comprising a diene- or dienophile, reaction of that dimedone derivative with an oligomer (optionally bound to a solid phase) to provide modified oligomers, the reaction of the diene- or dienophile modified oligomers with a diene or dienophile, which is immobilized on a solid support under conditions such that the inverse electron demand DA reaction occurs to form a diene/dienophile pair for the immobilization and separation (purification) of the full-length oligomer molecules from shorter synthesized molecules and residual monomers.

In a preferred embodiment the method comprises the step of contacting an electron-rich dienophile with an electron-poor diene.

In a further preferred embodiment, the synthesized oligomers are bound to a solid phase through a cleavable linkage before they react with the dimedone derivative. Thus, in certain embodiments, the method includes the further step of releasing the "oligomer-dimedone derivative product" (= modified oligomer) from the solid phase by cleaving the cleavable linkage before the inverse Diels Alder reaction is carried out.

The synthesis of the modified oligomers is based on the conditions as described in Brandt, O. et al., 2003, Nucleic Acids Res., 31: e119 (see, e.g. page 2, right column, second paragraph) and Pipkom, R. et al., J. Peptide Res., 2002, 59: 105-114 (see, e.g., page 107, bridging paragraph), whereas either a diene or a dienophile may be introduced as the last monomer for the modification.

The Oligomers may be automatically synthesized on standard synthesizers as already known in the art, e.g. in all kind of micro well formats (e.g. columns, cartridges, 96-well format, 384-well format or 1536-well format).

In the following a preferred embodiment of the present invention is described in more detail to illustrate the invention. This description, however, shall hot be interpreted as any limitation.

### Step 1:

Initially, the surfaces of a solid support (e.g. glass slides) may be derivatized by incubation with the free acid of the diene or dienophile and with any suitable type of coupling reagent in a solvent for 10 - 50 min, preferably 20 - 40 min. The free acid of the diene or the dienophile may be utilized at concentrations ranging from 0.01-0.1 mol/l, preferably 0.05 - 0.07 mol/l, more preferably 0.06 mol/l. The coupling reagents may be utilized at concentrations ranging from 0.01 - 0.15 mol/l, preferably, 0.05 - 0.1 mol/l, more preferably 0.06 - 0.08 mol/l. Afterwards, the solid support may be washed with any suitable type of solvent or a combination thereof. In a certain embodiment of the invention, the washing step is performed in an ultrasonic bath.

### Step 2:

The synthesis of PNAs is generally performed as described in Brandt, O. et al., 2003, Nucleic Acids Res., 31: e119 (see, e.g. page 2, right column, second paragraph). The synthesis of peptides is generally performed as described in Pipkom, R. et al., J. Peptide Res., 2002, 59: 105-114 (see, e.g. page 107, bridging paragraph). In both syntheses commercially available solid phase synthesizers and for protection standard Fmoc chemistry is used. In each synthesis the last monomer is a diene or dienophile.

Full-length modified oligomers are cleaved from the resin on which the oligomer synthesis took place at room temperature with 10 - 100 µl of a cleavage mixture consisting of either a strong carboxylic acid at concentrations ranging from 75-95% and a scavenger at concentrations ranging from 1 - 10%, preferably 5%. In a particular preferred embodiment the cleavage mixture contains trifluoroacetic acid (TFA) at concentrations ranging from 80 - 95%, more preferably 95%, and as the scavenger triisopropylsilane (TIS) at concentrations ranging from 5 - 10%, more preferably 5%.

Subsequently, the oligomers may be processed by precipitation with an ice-cold solvent and dissolution in water and stored at 4°C. In a particular preferred embodiment, the released oligomers may be precipitated in ice-cold diethyl ether, at least once, and after evaporation of the remaining ether, dissolved in water and stored at 4°C.

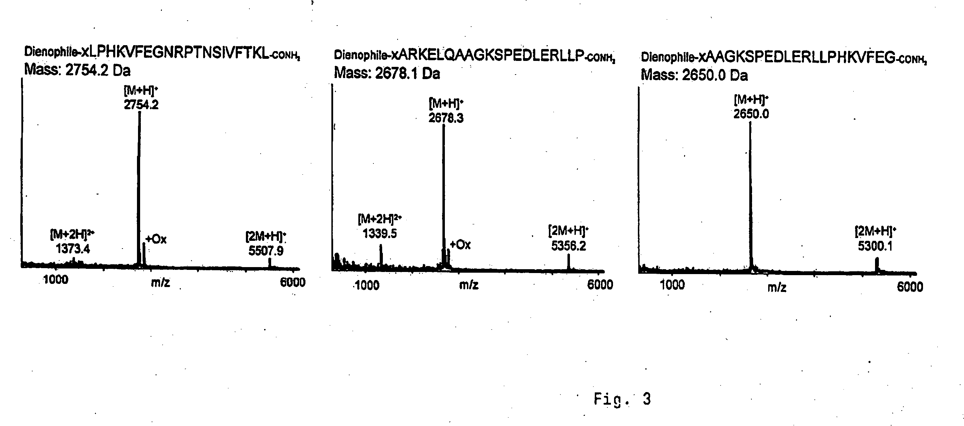

For a quality control, aliquots of the processed oligomers may be analysed with standard MALDI-TOF mass spectrometry (Fig. 3), based on the conditions as described in Brandt, O. et al., 2003, Nucleic Acids Res., 31: e119 (see, e.g. page 3, left column, second paragraph) and Pipkom, R. et al., J. Peptide Res., 2002, 59: 105-114 (see, e.g., page 108, right column, second paragraph).

### Step 3:

Following, the diene- or dienophile modified oligomers, synthesized as described above, may be spotted with a commercially available plotter on the modified solid support of Step 1 at concentrations ranging from 5 - 200 µM, using at each position a specified volume. Depending on the solid support employed, different spotting buffers may be used, e.g. betaine buffer with a pH of 7.0 - 7.5, BW buffer consisting of 10 mM. Tris-HCl, 1 mM EDTA and 2 mM NaCl with a pH of 7.5, 3 x PBS (phosphate buffered saline) - 0.25% dextran buffer with a pH of 7.0, and the like.

The invention is further described with reference to the attached figures:
- Fig. 1: shows the reaction for the purification of peptides (and PNAs) via the inverse Diels-Alder reaction. The dienophil is coupled via the dimedon to the terminal amino function of a peptide. After cleavage of the peptide from the synthesis resin the crude product is reacted with a tetrazin bound to a solid support. Here only full length products can be bound to the solid support via the inverse Diels Alder reaction and all residual products may be removed. The purified peptide is cleaved from the solid phase by aqueous hydrazine.

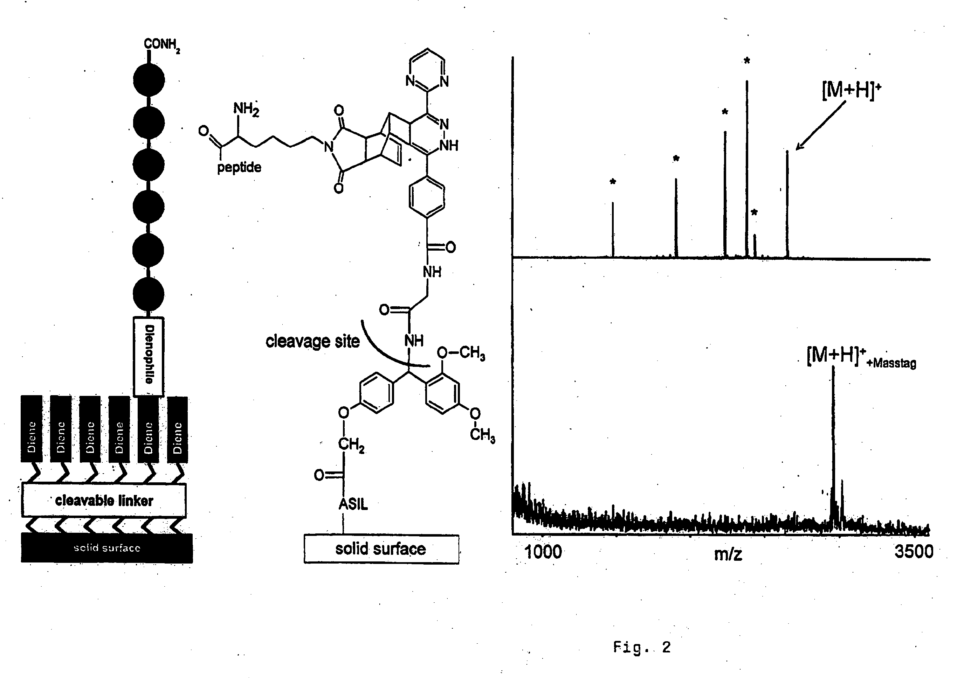

- Fig. 2:: shows the principle of the simultaneous binding and purification The surface of a solid surface is modified by binding a diene or dienophile via a linker. A PNA or peptide is carrying as last element a dienophile or diene. When spotting the modified peptide/PNA on the modified solid support surface, the dienophile on the one side and diene on the other side will react with each other via an inverse electron demand DA reaction and, thus, bind the peptide/PNA onto the solid support. All unbound contaminations or side products (e.g. shorter peptide/PNA sequences) may be removed by washing.
- Fig. 3:: Quality control of peptide synthesis. Mass spectra data of oligomer dienophile-xLPHKVFEGNRPTNSIVFTKL-CONH₃ ([M+H]⁺, *m*/*z* = 2754.2), dienophile-xARKELQAAGKSPEDLE-RLLP-CONH₃ ([M+H]⁺, *m*/*z* = 2678.1) and dienophile-xAAGKSPEDLERLLPHKVFEG-CONH₃ ([M+H]⁺, *m*/*z* = 2650.0).

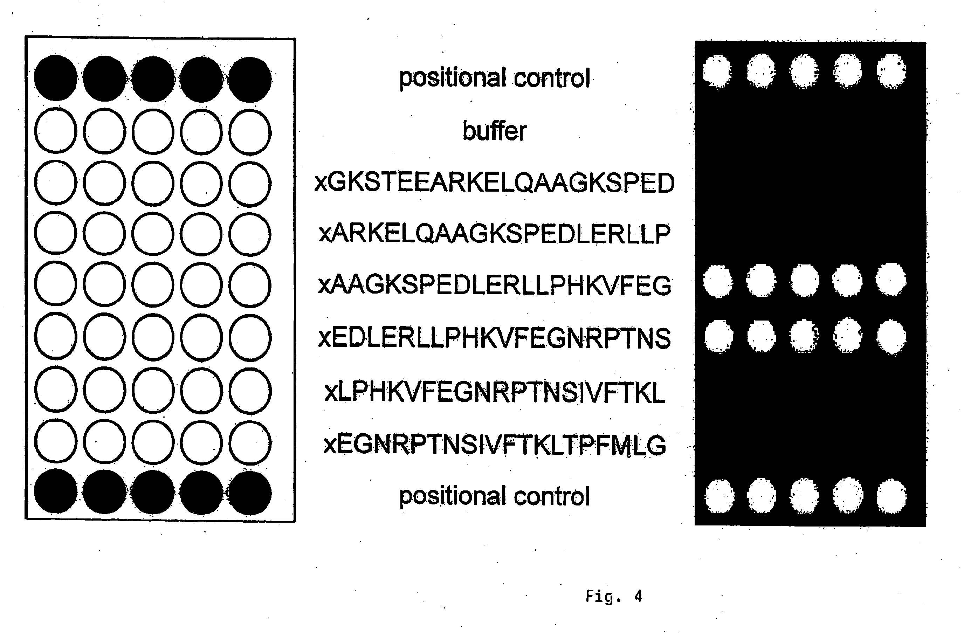

- Fig. 4:: Selective binding of full-length peptide molecules. Results obtained after the peptides xGKSTEEARKELQAAGKSPED, xARKELQAAGKSPE-DLERLLP, xAAGKSPELERLLPHKVFEG, xEDLERLLPHKVFEGNRPT-NS, xLPHKVFEGNRPTNSIVFTKL, xEGNRPTNSIVFTKLTPFMLG were hybridised at saturating conditions. Left: spot layout Middle: Sequences of the spotted peptides Right: incubated and read-out microarray (in inverse colours)
- Fig. 5:: Use of a dimedon-dienophil derivative modified with glycine for the solid phase peptide synthesis

The invention is further illustrated by the following non-limiting examples.

### Example 1: General Methods

### N-substituted amino acids

10 mmol of the Reppe-anhydride (Tetracyclo-[5.4.2.^{1,7}·O^{2,6}·O^{8,11}]3,5-dioxo-4-aza-9,12-tridecadien; Wiessler et al., Int. J. Med. Sci 2010, 7(4), pp. 213-223) were kept with 10 mmol of the respective acid in 20 ml of a 1:1 mixture DMF/Pyridin for 8 h at 80°C. Then the solution was reduced until a dry residue has been obtained. This residue was diluted in chloroform and washed with diluted acid and water. After drying with Na-sulfate it was filtered and dried. The dried residue was recrystallized. Yield: 70-90%.
Glycine: Recrystall: MeOH/Water 50/30, mass: calc. 259.1, found. 258.2
4-Amino-butyric acid: Recrystall.: Acetic Acid Ester, mass: calc. 287.1, found 288.1
5-Amino-valerianic acid: Recrystall.: Acetic Acid Ester, mass calc. 301.1
6-Amino-hexanic acid: Recrystall.: Acetic Acid Ester, mass: calc 315.2, found 314.4

### Dimedon Derivative

5 mmol of the respective acid were stirred together with 5 mmol dimedon (700 mg), 5 mmol DCC (1,03 gr) und 5 mmol dimethyl amino pyridine (610 mg) in 30 ml DMF at RT for 36 h. The resulting precipitate was filtered and the solution was reduced in vaccuo. It was diluted in acetic acid ester and washed with saturated bicarbonate solution and water to remove the dimedon. The organic phase was dried with Na-sulfate und the solution reduced. The mostly solid residue was recrystallized.
4-Amino-butyric acid: Recrystall.: Methanol, mass: calc. 409.2, found 408.4
5-Amino-valerianic acid: Recrystall.: Acetic Acid Ester, mass: calc: 423.2, found: 422.3
6-Amino-hexanic acid: Recrystall.: Methanol, mass: calc 437.2, found 436.2

### Example 2: Binding of the dienophile to peptides

After the solid phase synthesis (0.4 µmol resin load) the crude peptide bound at the resin was reacted with 100 µl of the dimedon derivative of Example 1(0.3 M in NMP) overnight. Unreacted dienophile was removed by washing with 6x60 µl DMF, 6x60 µl EtOH and 6x60 µl DCE. The cleavage of the dienophile-modified peptides from the synthesis resin was done with 95%TFA/5%TIS.

### Example 3: Production of tetrazin-modified surfaces

100 mg of a TentaGel S-NH₂ resin with a NH₂-load of 0.25 µmol/g was shaked with 0.5 ml of the acid chloride of tetrazine (0.15 M in NMP) over night at RT. The unbound tetrazine was removed by washing with 10x1.5 ml NMP, 2×1,5 ml EtOH and 2x1,5 ml DCM. The thus produced resin was kept at 4°C.

### Example 4: Binding of the dienophile-modified peptides to the tetrazine surface

0.2 µmol of a dienophile-modified crude peptide mixture was given in 50 µl 3xPBS/2M betaine (or in a TFA/acetonitrile/water mixture directly used) and 10 mg of the modified TentaGel S-NH₂ resin was added. The reaction took place overnight at RT.

### Example 5: Removal of impurities and cleavage from the surface

The resin was washed each with 500 µl solution to remove all impurities: 3xH₂O, 3xACN/H₂O 1:1 (v/v), 3xACN, 3xH₂O. To cleave the peptide from the surface the TentaGel was incubated with 10 µl 5% hydrazine/H₂O for 30 min and then washed with 25 µl 25% ACN/H₂O. The unified solutions were reduced until a dry residue was left and then diluted in 10 µl H₂O for mass spectroscopy.

### Example 6: Methods of synthesis of dienophile-modified peptides for array production

The synthesis of the peptides is performed in microwell plates according to standard protocols, whereas a dienophile or a dienophile derivative was introduced as the last monomer.

After the synthesis products were cleaved at room temperature with 10 µl per well of a cleavage mixture consisting of 95% TFA and 5% TIS (triisopropysilane) for 4 x 30 min. The cleaved peptides are then eluted with 2 x 40 µl TFA into another microtiter plate and precipitated with 800 µl ice-cold diethylether and dissolved in 50 µl water.

The quality control was performed via MALDI-TOF mass spectrometry (Fig. 3), wherein all spectra showed the [M+H]⁺-peak of the fully synthesized peptide and the peak of its dimer [M+H]²⁺, whereas the [M+2H]²⁺-peak shows the double loaded product.

The synthesized peptides are those of the known glucose-6-phoshate-isomerase (GPI) epitope whereas overlapping peptide sequences have been synthesized to make sure that in the following Example 11 the GPI epitope and non-binding regions of the protein could be discriminated.

### Example 7: Process for the manufacture of peptide arrays

The surfaces of glass-slides were derivatized by incubation with 16.6 mg (0.06 mmol) of the free acid of 1,2,4,5-tetrazine with 12.5 µl (0.08 mmol) di-isopropyl-carbodiimid (DIC) and 8.2 mg (0.06 mmol) 7-aza-1-hydroxybenzotriazol (HOAt) in 300 µl DMSO. After the reaction has been finished, the glass-slides were washed with 3 x 50 µl DMSO and 3 x 50 µl ml dichloroethane in an ultrasonic bath (each step 3 min).

Dienophile-modified peptides, synthesized as described in Example 6, were spotted in 5 replicates, respectively, onto the glass-slides at a concentration of 100 pmol/µl, using at each position 330 pl. The spot distance was 300 µm. As spotting buffer, 3 x PBS - 0.25% dextran buffer with a pH of 7.0 has been used. Spotting was performed with a NanoPlotter (Gesim, Großerkmannsdorf, Germany). After spotting, the glass slides were incubated at room temperature overnight and then washed with 10% DMSO - water, DMSO, water, ACN - water (1:1 mixture) and water.

### Example 8: Epitope mapping

Epitope mapping is the process for identification and characterization the minimum molecular structures, that are able to be recognized by the immune system, mainly T and B cells. This ability is based on the antibody's specific recognition of epitopes, i.e. the sites of the antigen to which antibodies bind. By selection from a phage antibody library, however, one might obtain a few hundred antibodies that bind the antigen. Epitope mapping might group the antibodies into classes that do not compete so that one or two members of each class can be tested for bioactivity. Thus, it is an important step in characterizing antibodies that bind a potential therapeutic target (Ladner, R. C., 2007, Biotechnol. Gen. Engin. Rev., 24: 1-30).

Epitope mapping may be performed with mono- or polyclonal antibodies either by dissecting the antigens into overlapping polypeptides in the form of recombinantly expressed fusion proteins or by synthesizing overlapping short or oligo-peptides or by a combination of both methods or by the generation of random gene fragments of approximately 20-200 bp length and cloning these into phages (Petersen, G. et al., 1995, Mol. Gen. Genet., 249: 425-431).

Before using the peptide array, as prepared in Example 7, for epitope mapping, it was washed with 1 x PBS (phosphate buffered saline), pH 7.4 and then blocked by incubation with 1 x PBS, pH 7.4, containing 1% skim milk powder at room temperature for 2 h. Then, it was incubated overnight with KBN mouse serum (KBN mouse serum is derived from a mouse having arthritis and contains antibodies against glucose-6-phosphate-isomerase; Korganow A.-S. et al., 1999, Immunity, 10: 451-461) in 1% milk powder/1 x PBS, pH 7,4.

After washing with 0.1% Tween20/1 x PBS, pH 7.4 and 1 x PBS, pH 7.4, an incubation followed with a dye-labelled anti-mouse-antibodies in 1% skim milk powder/1 x PBS, pH 7.4 at room temperature for 2 hours.

After additional washing steps with 0.1% Tween20/1 x PBS, pH 7.4, 1 x PBS, pH 7.4 and water the signals were automatically detected with a laser scanner (Scan Array 3000, Packard, Billerica, USA).

As shown in Fig. 4, the synthetically produced dienophile modified peptides have been successfully bound via an inverse electron demand DA reaction to the diene-derivatized solid support and show only signals for the epitope but not for the negative control.

### Example 9: Effectivity of the purification by selective binding of the modified pepetides to a surface

### - Introduction of a cleavable linker

A Fmoc-rink linker was coupled to a surface (20x20mm silica wafer) for four hours as follows: A mixture of (1:1:1) (v/v/v) of 0.4 M DIC, 0.3 M HOAt und 0.3 M Fmoc-rink linker in NMP was activated for 3 min. Then 20 µl of this mixture were given on a silanisized surface, covered with a glass cover and coupled for four hours at RT. After two washing steps with DMF the free amino groups on the surface were acetylized. For this the surfaces were incubated twice (3 min und 5 min) with a solution consisting of 240 µl acetic acid anhydride und 240 µl 2,6-Lutidine in 20 ml DMF. After a washing step with DMF the Fmoc-groups were cleaved by two incubations (3 min und 5 min) in 20% piperidine in DMF. After washing with 3xDMF and 2xEtOH the surfaces were dried und kept at 4°C.

### - Modification of the cleavable linker modified surface with a tetrazine

For the surface derivatization of Rink-linker modified surfaces (Example 2) 1,66mg (0,006mmol) of the free acid of the tetrazine were brought with 1,25µl DIC (0,008mmol) and 0,82mg (0,006mmol) HOAt in 30µl DMSO onto the surface. After copmpleting of the reaction the slides were washed with 3x15ml DMSO and 3x15ml Dichlorethane in an ultra sonic bath (each 3min).

### - Cleavage of the peptide from the surface

The covalently bound peptides were cleaved from the surface by a 2 hour incubation with 10 µl 95% TFA/5% TIS. After the TFA was evaporated the cleaved full length product could be diluted in water and analyzed by mass spectrocopy (Fig 2 right).

## Claims

1. A dimedon derivative selected from the group consisting of:

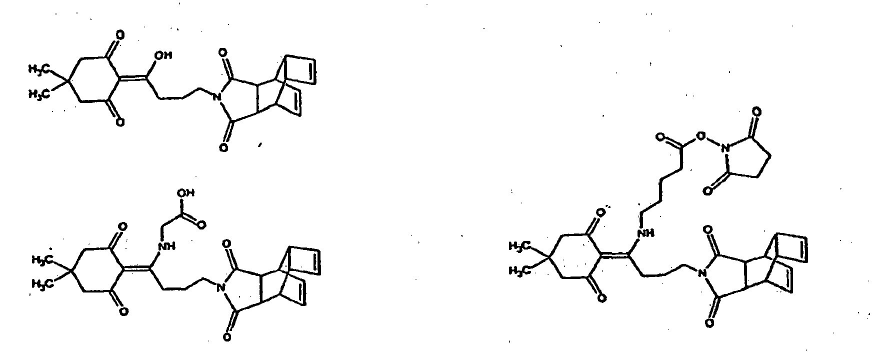

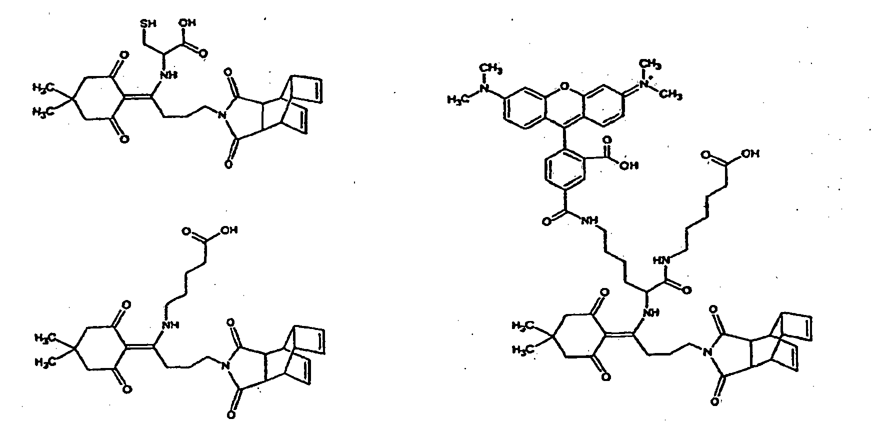

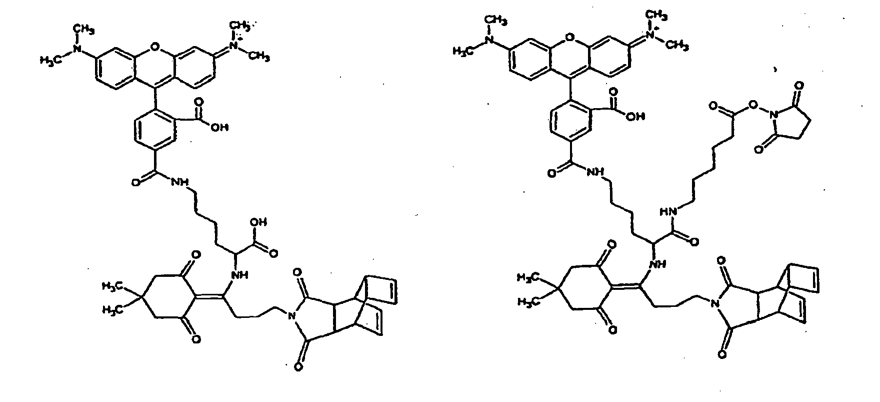

2. A method for the production and purification of peptide nucleic acids and peptide oligomers on a solid support, comprising the steps of:
- modifying the solid support with a diene or dienophile as a first binding partner on its surface to obtain a modified solid support;
- reacting the surface-immobilized diene or dienophile via an inverse demand Diels Alder reaction with a peptide oligomer carrying a dimedon derivative as defined in claim 1 as a second binding partner

3. The method of claim 2, wherein said solid support is selected from glassware; polymeric films or membranes, semiconductors, nanotubes, chitin, chitosan, ceramics, hybridpolymers or nanocomposites.

4. The method of claim 2 or 3, wherein the diene or dienophile is immobilized on the solid support through a direct bond, a linker or spacer arm.

5. The method of any of claims 2 to 4, wherein the peptide oligomer is a biomolecule with an amide backbone, which consists of a number of monomer units in the range of 5 - 100 monomers.

6. The method of claim 5, wherein the peptide oligomer is peptide nucleic acids (PNAs) or an oligopeptide consisting of 2 -10 amino acids.

7. The method of any of claims 2 to 6, wherein the method further contains the step of cleaving the peptide oligomer with aqueous hydrazine from the Diels Alder product that is bound to the solid support to obtain a purified peptide oligomer.

## Patentansprüche

1. Dimedonderivat, ausgewählt aus der Gruppe bestehend aus:

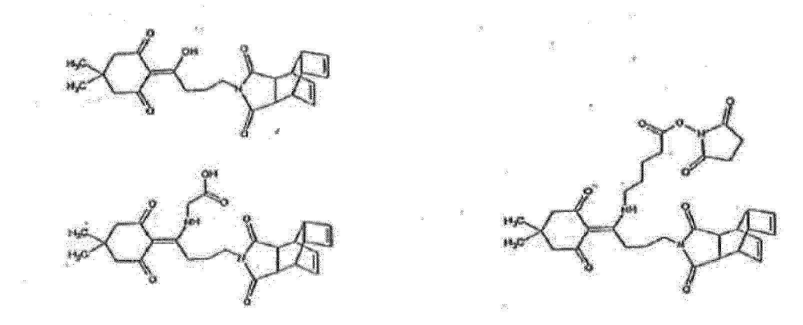

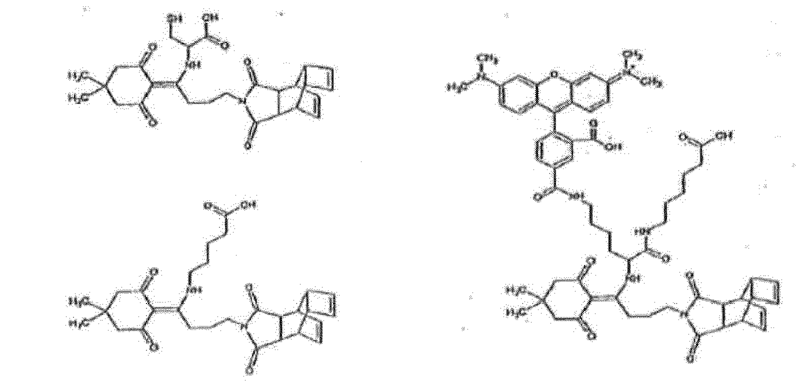

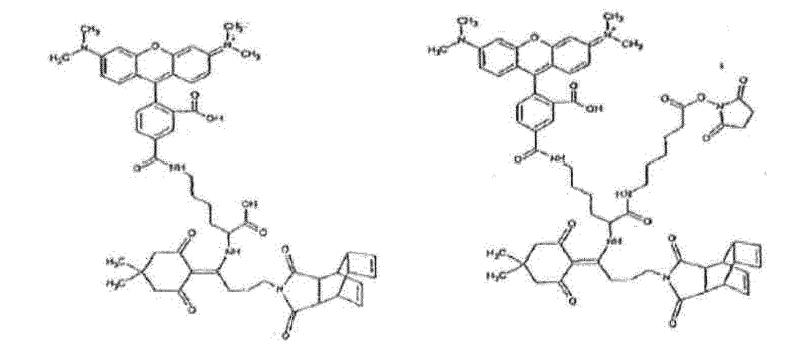

2. Verfahren zur Herstellung und Reinigung von Peptidnukleinsäuren und Peptidoligomeren auf einem festen Träger, umfassend die Schritte:
- Modifizieren des festen Trägers mit einem Dien oder Dienophil als erstem Bindungspartner auf der Oberfläche, um einen modifizierten festen Träger zu erhalten;
- Reagieren des oberflächenimmobilisierten Diens oder Dienophils über eine Diels-Alder-Reaktion mit inversem Bedarf mit einem Peptidoligomer, das ein Dimedonderivat nach Anspruch 1 trägt, als zweitem Bindungspartner.

3. Verfahren nach Anspruch 2, wobei der feste Träger aus Glas, polymeren Filmen oder Membranen, Halbleitern, Nanoröhrchen, Chitin, Chitosan, Keramik, Hybridpolymeren oder Nanoverbundstoffen ausgewählt wird.

4. Verfahren nach Anspruch 2 oder 3, wobei das Dien oder Dienophil auf dem festen Träger durch eine direkte Bindung, einen Linker oder Spacer-Arm immobilisiert wird.

5. Verfahren nach einem der Ansprüche 2 bis 4, wobei das Peptidoligomer ein Biomolekül mit einem Amidgerüst ist, das aus einer Anzahl von Monomereinheiten im Bereich von 5 - 100 Monomeren besteht.

6. Verfahren nach Anspruch 5, wobei es sich bei dem Peptidoligomer um Peptidnukleinsäuren (PNAs) oder ein Oligopeptid handelt, das aus 2 - 10 Aminosäuren besteht.

7. Verfahren nach einem der Ansprüche 2 bis 6, wobei das Verfahren weiter den Schritt des Spaltens des Peptidoligomers mit wässrigem Hydrazin von dem Diels-Alder-Produkt umfasst, das an den festen Träger gebunden ist, um ein gereinigtes Peptidoligomer zu erhalten.

## Revendications

1. Dérivé de dimédon choisi parmi le groupe composé de:

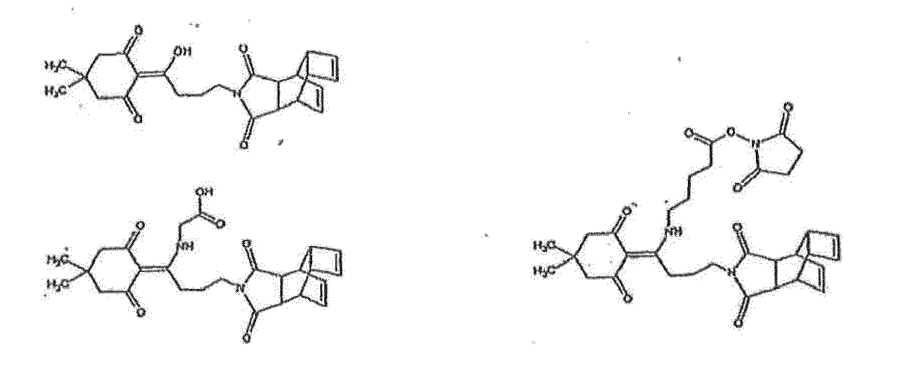

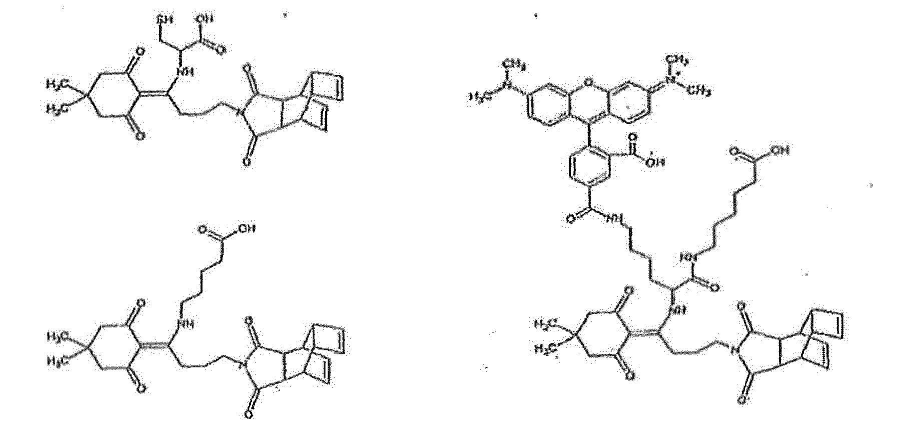

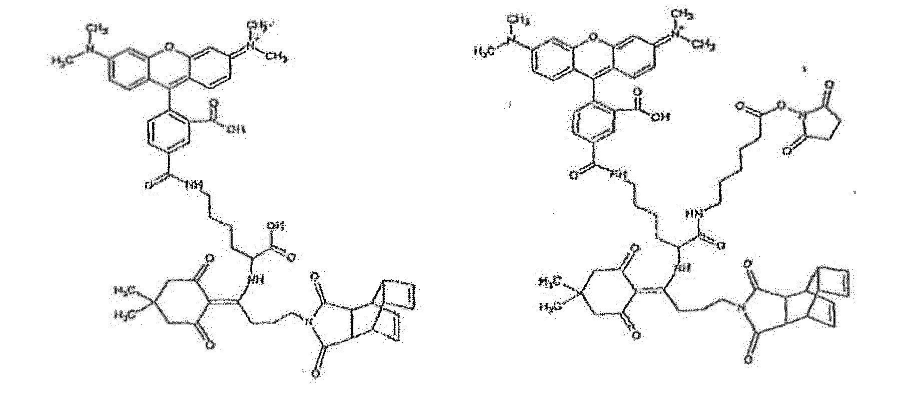

2. Procédé de production et de purification d'acides nucléiques peptidiques et d'oligomères de peptide sur un support solide, comprenant les étapes consistant à:
- modifier le support solide avec un diène ou diénophile comme premier partenaire de liaison sur sa surface, pour obtenir un support solide modifié;
- faire réagir le diène ou diénophile immobilisé sur la surface par une réaction de Diels Alder à demande inverse avec un oligomère de peptide portant un dérivé de dimédon tel que défini à la revendication 1 comme deuxième partenaire de liaison.

3. Procédé selon la revendication 2, dans lequel ledit support solide est sélectionné parmi le verre, les films ou membranes polymères, les semiconducteurs, les nanotubes, la chitine, le chitosan, les céramiques, polymères hybrides ou les nanocomposites.

4. Procédé selon la revendication 2 ou 3, dans lequel le diène ou diénophile est immobilisé sur le support solide par une liaison directe, un bras de liaison ou de séparation.

5. Procédé selon l'une quelconque des revendications 2 à 4, dans lequel l'oligomère de peptide est une biomolécule avec un squelette amide qui se compose d'un certain nombre d'unités monomères dans la gamme de 5 à 100 monomères.

6. Procédé selon la revendication 5, dans lequel l'oligomère de peptide est des acides nucléiques peptidiques (PNA) ou un oligopeptide composé de 2 à 10 acides aminés.

7. Procédé selon l'une quelconque des revendications 2 à 6, dans lequel le procédé comprend par ailleurs l'étape de clivage de l'oligomère de peptide par de l'hydrazine aqueuse à partir du produit de Diels Aider qui est lié au support solide, pour obtenir un oligomère de peptide purifié.
